# EUROPEAN PATENT SPECIFICATION

(11) **EP 4 245 336 B1**
(45) Date of publication and mention of the grant of the patent: **18.11.2026**
(21) Application number: 22305302.6
(22) Date of filing: 15.03.2022
(51) Int. Cl.: A61M 11/00, A61M 5/315

(54) **INJECTION DEVICE COMPRISING A SETTING DOSE MEMBER**
INJEKTIONSVORRICHTUNG MIT EINEM DOSISEINSTELLELEMENT
DISPOSITIF D'INJECTION COMPRENANT UN ÉLÉMENT DE RÉGLAGE DE DOSE

(43) Date of publication of application: 20.09.2023
(73) Proprietor: Becton Dickinson France, 38800 Le Pont-de-Claix (FR)
(72) Inventor: HUANG, Longxiang, Suzhou, Jiangsu 215000 (CN); HUANG, Wilson, Suzhou, Jiangsu 215000 (CN); YAN, Bo, Shanghai 200336 (CN)
(74) Representative: Germain Maureau

(56) References cited:
- WO-A1-2021/144668
- DE-B- 1 006 589
- US-A1- 2021 330 887

## Description

### Technical field

The invention relates to an injection device, for example a medical injection device such as a syringe or the like.

### Background

Today medical injection devices - such as prefilled syringes - intended to contain and deliver several doses of the same medical injection fluid are not always convenient for delivering accurate doses, for example small doses (e.g. 1ml). Moreover, expelling the potential air bubble contained in the medical injection device may require a high activation force, and thus decrease the dose accuracy of the fluid to be injected. For example, the injection devices disclosed by US 5 601 077, US 9 579 465, US 2021/0196906, DE 10 06 589 B, US 2021/330887 A1, WO 2021/144668 A1 may be not so easy to handle and or not so accurate. In addition, the structure of such devices may be very complex. Therefore, a need exists to provide a reliable and user friendly injection device for delivering accurate doses and/or showing a simple structure.

### Summary

The invention is defined in the independent claims 1 and 11. An embodiment relates to an injection device including a barrel extending along an axis and defining a reservoir, the barrel comprising a proximal end and a distal end having an outlet, a plunger rod received within the barrel via the proximal end and configured to move along the axis relative to the barrel, the plunger rod comprising N radial projections successively disposed at different locations along the axis, N being an integer equal or greater than 2, a dose setting member configured to be mounted onto the barrel and having N portions configured to sequentially abut axially against the N radial projections, respectively, when moving the plunger rod axially in a first direction (or distal direction) oriented from the proximal end toward the distal end, the dose setting member being configured to be moved relative to the barrel between N positions, wherein the n^{th} radial projection axially faces the n^{th} portion in the n^{th} position, n being an integer lying between 1 and N.

An axial direction is the direction defined by the axis of the barrel. A radial direction is a direction perpendicular to the axis. A circumferential or azimuthal direction corresponds to the direction describing a ring around the axial direction. The three directions axial, radial and azimuthal correspond respectively to the directions defined by the height, the radius and the angle in a cylindrical coordinate system. In addition, upstream and downstream may be defined with respect to the moving direction of the plunger rod to expel/deliver the content of the reservoir, when moved in the first direction.

A distal side is the side of the injection device from which the content of the reservoir is expelled/delivered, and a proximal side is the side opposite to the distal end along the axis, which is usually handled by the user during injection.

Each n^{th} radial projection may comprise a single or several protrusions extending radially. Each n^{th} portion may comprise a single or several abutment/shoulder parts. A single abutment/shoulder part of the n^{th} portion may be configured to cooperate with a single or several protrusions of the n^{th} radial projection. Several abutment/shoulder parts of the n^{th} portion may be configured to cooperate with a single or several protrusions of the n^{th} radial projection.

The radial projection are successively disposed, i.e. arranged/ordered from the 1^{st} radial projection up to N^{th} the radial projection along the axis (when the plunger rod is mounted within the reservoir). In other words, the n^{th} radial projection is between the (n-1)^{th} radial projection and the (n+1)^{th} radial projection.

The portions may be successively arranged, but not necessarily.

The N portions are configured to sequentially abut axially against the N projections, respectively, i.e. the 1^{st} projection is configured to axially abut against the 1^{st} radial projection, the 2^{nd} projection is configured to axially abut against the 2^{nd} radial projection, etc.

For example, the movement of the dose setting member between two successive positions may be a translation, a rotation, a combination thereof, etc.

The plunger rod may comprise, 2, 3, 4, etc. radial projections and the dose setting member may comprise the same number of portions, i.e. 2, 3, 4, etc., respectively, and may be move between the same number of positions, i.e. 2, 3, 4, etc. respectively. In the 1^{st} position, the 1^{st} radial projection axially face the 1^{st} portion, in the 2^{nd} position, the 2^{nd} radial projection axially face the 2^{nd} portion, etc..

During a single total injection stroke of the plunger rod (i.e. in the first direction), the dose setting member is successively placed in the 1^{st}, 2^{nd}, 3^{rd} (if any), etc. position, so that the 1^{st}, 2^{nd}, 3^{rd} (if any), etc. radial projection successively axially face the 1^{st}, 2^{nd}, 3^{rd} (if any), etc. portion. The partial stroke from the initial position of the plunger rod up to the abutment of the 1^{st} radial projection with the 1^{st} portion correspond to a first dose (which may be a priming dose), the partial stroke from the 1^{st} radial projection up to the abutment of the 2^{nd} radial projection with the 2^{nd} portion correspond to a second dose, etc.

By moving the dose setting member between the different positions, a predetermined dose is pre-set. For example, in the n^{th} position, the injection device is pre-set for an n^{th} injection.

In other words, the dose setting member is firstly placed in the 1^{st} position. Then a first delivery (or priming) may be carried out by moving the plunger rod from its initial position (the reservoir being full, not injection/delivery carried out yet), up to abutting the 1^{st} radial projection with the 1^{st} portion. Subsequently, the dose setting member may be moved in the 2^{nd} position. Then a second delivery (or first injection) may be carried out by moving the plunger rod from its former position (corresponding to the axial position of the plunger rod at the end of the first injection/delivery, i.e. when the 1^{st} radial projection abuts the 1^{st} portion), up to abutting the 2^{nd} radial projection with the 2^{nd} portion. Etc.

Due to the configuration of the injection device, the user may easily and reliably set a dose by simply moving of the dose setting member relative to the reservoir while the axial abutment of the radial projections with the portion ensure a reliable stopping means. In addition, the configuration of the radial projections and of the portions may provide a simple structure.

In some embodiments, the injection device may be configured so that the n^{th} portion is able to abut only against the n^{th} radial projection.

In some embodiments, the dose setting member may be configured to adopt at least one N+1 position, wherein the plunger rod is axially free relative to the dose setting member.

For example, when the dose setting member is placed in the N+1 position, the plunger rod may be moved for carrying out a (N+1)^{th} injection after the N^{th} injection (when the N^{th} radial projection abuts against the N^{th} portion) or in second direction (or proximal direction), opposed to the first direction in order to suck/pump a substance to be injected, for example a liquid but not necessarily, within the reservoir, via the outlet. Alternatively the medical injection device may be prefilled with the substance to be injected. The injection device may comprise a single N+1 position or several N+1 positions, for example a first N+1 position in order to suck/pump a substance to be injected, and a second N+1 position in order to carry out a (N+1)^{th} injection. For example, the first N+1 position may be an initial position before the 1^{st} positon (in such a case the first N+1 position may be also considered as a 0 position) and the second N+1 position may be the last position, after the N^{th} position.

In some embodiments, the dose setting member may be removably mounted onto the barrel.

With such a configuration, a N+1^{th} dose may be set, corresponding to the last stroke of the plunger rod from the axial position of the plunger rod at the end of the N^{th} injection, i.e. when the N^{th} radial projection abuts the N^{th} portion, up to an abutment of the plunger rod with a part of the barrel, for example a bottom of the reservoir defined in fluid communication with the outlet.

In some embodiments, the dose setting member may be configured to be radially moved relative to the barrel between N positions.

Such a radial movement may be easy to carried out, and allows a reliable checking for the user in order to be sure that the appropriate dose is set.

In some embodiments, the dose setting member may comprise a notch extending radially and radially opened, considered when the dose setting member is mounted onto the barrel, and configured to receive the barrel.

When the dose setting member is mounted onto the barrel, the notch may extend in a plane perpendicular to the axis. The walls extending around the notch or defining the notch, may define a general U-shape. The barrel and the plunger rod may be introduced within the notch via the aperture of the U-shape, i.e. the open top of the "U", and extend transversally to the plane in which the "U-shape" extends.

In some embodiments, the notch may extend along a second axis, the N portions being successively disposed at different locations along the second axis.

When the dose setting portion is mounted onto the barrel, the second axis may extend radially. The portions may be arranged/ordered from the 1^{st} portion up to N^{th} the portion along the second axis. In other words, the n^{th} portion may be between the (n-1)^{th} portion and the (n+1)^{th} portion.

In some embodiments, a radial extension of the t^{th} radial projection may be greater than a radial extension of the (t-1)^{th} radial projection and lower than a radial extension of the (t+1)^{th} radial projection, if any, t being an integer lying between 2 and N.

In other words, the radial projections may be arranged in growing size from the 1^{st} to the N^{th} radial projection.

In some embodiment, all or part of the radial projection may have a radial extension smaller than a radius of the reservoir. This allows said radial projections to be inserted within the reservoir, into the barrel.

In some embodiments, each of the radial projections may have an annular shape having a radius, the radius of the t^{th} radial projection being greater than the radius of the (t-1)^{th} radial projection, and lower than the radius of the (t+1)^{th} radial projection, if any, and each of the portions defines an axial passage having a minimal radial length, the minimal radial length of the t^{th} portion being greater than minimal radial length of the (t-1)^{th} portion and lower than minimal radial length of the (t+1)^{th} portion, if any, t being an integer lying between 2 and N.

The annular shape of each radial projection may be continuous, i.e. ring shape, or discontinuous, e.g. a series of protrusions or ring portions arranged annularly or circumferentially. The radial projections may be arranged in growing size from the 1^{st} to the N^{th} radial projection.

The minimal radial length of the t^{th} portion may be greater than the diameter on the (t-1)^{th} radial projection and lower than the diameter of the t^{th} radial projection. The minimal radial length may correspond to the shorter distance between two facing walls delimiting the notch and extending along the second direction, at the t^{th} position.

In some embodiments, the N portions may be successively arranged and may globally have a stair shape, each portion forming a step of the stair shape.

In some embodiments, in the at least one N+1 position, the dose setting member may define an axial passage having a minimal radial length which is greater than the radius of the N^{th} radial projection.

In some embodiments, the (t-1)^{th} radial projection may be axially closer to the distal end than the t^{th} radial projection.

In some embodiments, the barrel may comprise a flange extending radially, the dose setting member being configured to be slidably mounted onto the flange.

The dose setting member may have one or several grooves receiving all or part of the flange. For example, the flange may form a rib slidably engaged within the groove(s) of the dose setting member. The flange may have any shape, e.g. annular shape, oblong or elliptical shape, rhomboid shape, square, rectangular or any polygonal shape with sharp or rounded angle, etc.

In some embodiments, the flange may comprise a guiding portion configured to slidably guide the dose setting member, and a retaining portion configured to maintain the dose setting member in any of the N positions.

The guiding portion may comprises two tongues extending radially in opposed directions. For example, when the dose setting member is mounted onto the barrel, the guiding portion may extend along the second axis of the notch and may radially guide the dose setting member in translation along the second axis.

For example, the retaining portion may cooperate by snap fitting with the dose setting member. For example, the retaining member may form a ratchet configured to be engaged with different complementary portions of the dose setting member.

In some embodiments, the injection device may be a medical syringe. The syringe may be disposable and/or prefilled.

In some embodiments, the injection device may comprise a spray nozzle, which may be removable.

In some embodiments, the injection device may comprise a vial adapter, which may be removable.

An embodiment also relates to a dose setting method for pre-setting a dose of an injection device before injection, the dose setting method comprising providing an injection device according to any one of the embodiments of the present disclosure, and moving the dose setting member in any one of the N positions so that a n^{th} radial projection axially face a n^{th} portion.

In some embodiments, the dose setting member may be moved to an n^{th} position in view of an n^{th} injection.

In some embodiments, the dose setting member may be removably mounted onto the barrel and the dose setting method may comprise removing the dose setting member for setting the last dose.

In some embodiments, the dose setting member may be configured to adopt at least one N+1 position, wherein the plunger rod is axially free relative to the dose setting member and the dose setting method may comprise, moving the dose setting member in one of the at least one N+1 position before setting the first dose for filling the injection device or after completion of the N^{th} dose to allow emptying the injection device.

The configurations according to any one of the above embodiments may take part in providing a reliable, user friendly and/or simple structure and method.

### Brief description of the drawings

The disclosure and its advantages can be better understood by reading the detailed description of various embodiments given as non-limiting examples. The description refers to the accompanying sheets of figures, in which:
Figure 1 shows an injection device;
Figure 2 shows a top view of the dose setting member;
Figure 3 shows a lateral view of the dose setting member;
Figure 4 shows another view of the dose setting member;
Figures 5A to 5G show different steps for setting a dose with the injection device;
Figures 6A and 6B show cross sectional views of the injection device; in section VI of figure 1,
Figure 7 shows a top view of the dose setting member according to a first variant;
Figure 8 shows a lateral view of the dose setting member according to the first variant;
Figure 9 shows another view of the dose setting member according to the first variant;
Figures 10A to 10G show different steps for setting a dose with the injection device according to the first variant;
Figures 11A to 11C show cross sectional views of the injection device according to the first variant, in a section similar to section VI;
Figure 12 shows a top view of the dose setting member according to a second variant;
Figure 13 shows a lateral view of the dose setting member according to the second variant;
Figure 14 shows another view of the dose setting member according to the second variant;
Figures 15A to 15G show different steps for setting a dose with the injection device according to the first variant;
Figures 16A to 16D show cross sectional views of the injection device according to the second variant, in a section similar to section VI.

### Detailed description

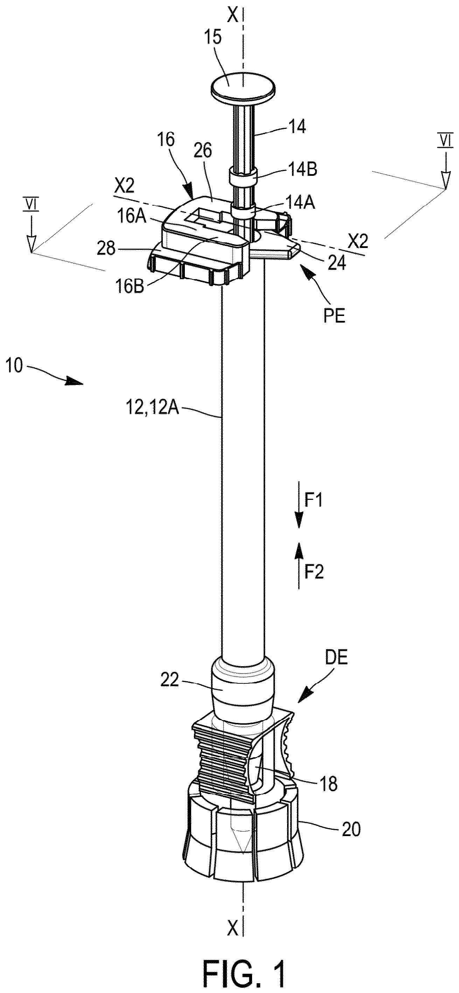

Figure 1 show an injection device 10 including a barrel 12, a plunger rod 14 and a dose setting member 16. In the present example, the injection device 10 is a medical injection device, for example a syringe. In this example, the injection device 10 comprises spray nozzle 18 and a removable vial adapter 20. For example, the injection device 10 may be prefilled with powder drug, liquid medical component or any other active component, such as medical drug, vaccines, etc.

The barrel 12 extends along an axis X and defines a reservoir 12A therein. The barrel 12 comprises a proximal end PE and a distal end DE. The distal end DE has an outlet 22. The spray nozzle 18 and the vial adapter 20 are disposed at the distal end DE. The spray nozzle 18 is in fluid connection with the reservoir 12A via the outlet 22. Alternatively to the spray nozzle 18, a needle may be attached to the distal end DE of the barrel in order to inject the medical component to a patient.

The distal side of the injection device 10 corresponds to the side of the distal end DE and the proximal side of the injection device 10 corresponds to the side of the proximal end PE.

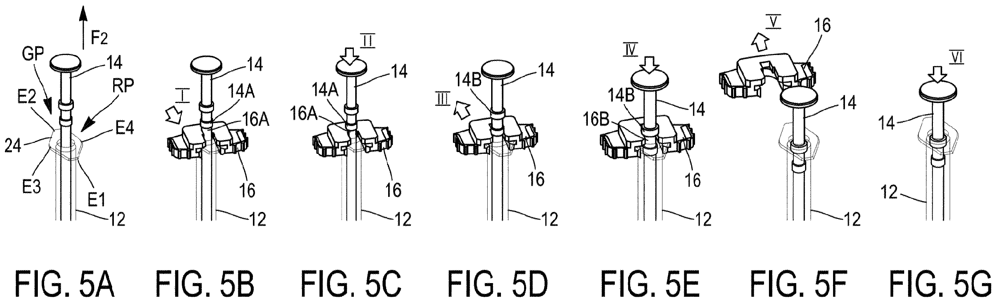

In the present example, the barrel 12 comprises a flange 24 extending radially, the flange 24 being disposed at the proximal end PE of the barrel 12. A seen in figure 5A, the flange 24 has a general rhomboid shape with rounded angles. In this example, the flange 24 comprises a guiding portion GP configured to slidably guide the dose setting member 16, and a retaining portion RP configured to maintain the dose setting member 16 in any of the N positions. The guiding portion GP comprises two tongues E1, E2 extending radially in opposed directions. The retaining portion RP comprises two radially opposed edges E3 and E4. The two tongues E1 and E2 extend along the larger diagonal of the rhomboid shape and the two edges E3 and E4 are disposed at the tow opposed summit joined by the smaller diagonal of the rhomboid shape. In the present example, the edges E3 and E4 from two ratchets configured to be engaged with different complementary portions of the dose setting member 10, described thereafter. In a variant, the flange may have any shape, for example a circular shape.

The plunger rod 14 is received within the barrel 12 via the proximal end PE and is configured to move along the axis X relative to the barrel 12. The plunger rod 14 comprises N radial projections 14A, 14B successively disposed at different locations along the axis X, N being an integer equal or greater than 2. In the present example N equals two (N=2). In this example, the plunger rod 14 comprises a pusher portion 15 at a proximal end thereof and a stopper (not shown) at a distal end thereof and disposed within the reservoir 3. For injection/spraying of the medical component contained in the barrel 12, the end user may apply his thumb on the pusher portion 15 of the plunger rod 14 in order to ease the injection/spraying.

In the present example, as shown in figure 1 and figures 5A to 5G, a radial extension of the 2^{nd} radial projection 14B is greater than a radial extension of the 1^{st} radial projection 14A. In other words, a radial extension of the distal-most radial projection 14B is greater than a radial extension of the proximal-most radial projection 14A. In the present example, the radial projections 14A, 14B have an annular shape, in this example a ring shape, having a radius, the radius of the 2^{nd} radial projection 14B (or distal-most radial projection) being greater than the radius of the 1^{st} radial projection 14A (or proximal-most radial projection). The 1^{st} radial projection 14A is axially closer to the distal end DE than the 2^{nd} radial projection 14B. In the present example, the radius of all the outer radial projections is smaller than the inner radius of the reservoir 12A, so as to be able to be inserted within the reservoir 12A, into the barrel 12.

The dose setting member 16 is configured to be mounted onto the barrel 12. In this example, the dose setting member 16 is configured to be slidably mounted onto the flange 24. The dose setting member 16 has N portions 16A, 16B configured to sequentially abut axially against the N radial projections 14A, 14B, respectively, when moving the plunger rod 14 axially in a first direction (or distal direction) F1 oriented from the proximal end PE toward the distal end DE. The dose setting member 16 is configured to move relative to the barrel 12 between N positions, wherein the n^{th} radial projection axially faces the n^{th} portion in the n^{th} position, n being an integer lying between 1 and N, i.e. in the present example between 1 and 2. In the present example, the dose setting member 16 is removably mounted onto the barrel (see in particular figures 5A and 5F). As shown in figures 5A to 5G, the dose setting member 16 is configured to radially move relative to the barrel 12 between N positions, in a direction perpendicular to the axis X.

The dose setting member 16 comprises a notch 100 extending radially and radially opened, considered when the dose setting member 16 is mounted onto the barrel 12, and configured to receive the barrel 12. The notch 100 extends along a second axis X2, the N portions being successively disposed at different locations along the second axis X2. The walls extending around the notch 100 define a general U-shape. The barrel 12 and the plunger rod 14 may be introduced within the notch 100 via the aperture of the U-shape, i.e. the open top of the "U", and extend transversally to the plane in which the "U-shape" extends (see figure 5B). When the dose setting portion 16 is mounted onto the barrel 12, the second axis X2 extends radially, perpendicularly to the axis X.

In the present example, the dose setting member 16 comprises a top portion 26, which may be a top plate, extending in a radial plane perpendicular to the axis X, considered when mounted onto the barrel 12. The top portion 26 is configured to be oriented toward the distal end of the plunger rod 14 (or in this example the flange 15 of the plunger rod 14). The lateral walls of the U-shaped notch 100 in the top portion 26 are configured to receive and cooperate with plunger rod 6. Two opposed shoulders are formed on both sides of the notch 100, at each portion 16A, 16B configured to axially abut against a radial projections 14A, 14B of the plunger rod 14. In the present example, portion 16A comprises two opposite shoulders (in this example radial shoulders) 16A1, 16A2, and portion 16B comprises two opposite shoulders (in this example radial shoulders) 16B1, 16B2. In this example, each of the portions 16A, 16B defines an axial passage having a minimal radial length, the minimal radial length RL2 of the 2^{nd} portion 16B being greater than minimal radial length RL1 of the 1^{st} portion 16A.The rest of the plunger rod 6 (i.e. the rod of the plunger rod, without the radial projections) is received within the notch 100 and is free to axially move relative to the dose setting member 16. The top portion 26 may comprises signs to indicate information about a dose at said position. In the present example, the portions 16A, 16B are successively arranged along the second axis X2, the last portion 16B being closer to the open top of the "U" than the first portion 16A.

In the present example, the dose setting member 16 comprise a lower portion 28, which may be a lower plate, extending in a radial plane perpendicular to the axis X and parallel to the second axis X2. The lower portion 28 is configured to be oriented toward the distal end DE of the barrel 12.The lateral walls of the U-shaped notch 100 in the lower portion 28 are configured to receive and cooperate with the barrel 12 (or a barrel body). The width, perpendicular to the second axis X2, of the notch 100 in the lower portion 28 is constant all along the second axis X2. This width may be greater than the maximum width of the notch 100 in the top portion 26.

The top portion 26 and the lower portion 28 are adjacent along the axis X (when mounted onto the barrel 12), and form together a same and single element. Two facing grooves 30 are arranged in the opposed lateral walls of the notch 100, axially between the top portion 26 and the lower portion 28. The grooves 30 extend along the second axis X2. An opening 38 is arranged between the two grooves 30, through which a tongue E1 or E2 of the flange 24 may extend, at least in part. The grooves 30 are configured to slidably receive at least in part the flange 24. Reliefs 32 are arranged in the grooves 30, in this example on the bottom of the grooves 30, and are configured to cooperate by snap fitting with the edges E3 and E4 of the flange 24. In other words, the reliefs 32 form different complementary portions configured to engage with the edges E3 and E4. The reliefs 32 are arranged by pair, the two members of the pair facing each other relative to the second axis X2. It the present example, each snap fitting position correspond to an n^{th} position of the dose setting member 16.

In the present example, the outer edges of the dose setting member 16 comprise a grip portion 34 having anti-slipping ribs 34A, to facilitate the handling of the dose setting member 16.

The dose setting member 16 may be made of polypropylene, polyester, polycarbonate, polypropylene, polyethylene, polyethylene terephthalate, acrylonitrile butadiene styrene, and preferably the dose setting member 16 may be made of polypropylene.

In the present example, the injection device 10 is configured so that the n^{th} portion is able to abut only against the n^{th} radial projection.

A dose setting method for pre-setting a dose of the injection device 10 before injection, comprising moving the dose setting member 16 in any one of the N positions so that an n^{th} radial projection axially face an n^{th} portion, is now described with reference to figures 5A to 5G.

In figure 5A, the injection device 10 is not provided with the dose setting member 16. A reconstitution of an injection fluid, may be carried out by mixing a prefilled powder and a fluid. As an alternative, an injection fluid may be pumped/sucked from an external vial (not shown) containing the injection fluid while the reservoir 12A is initially empty. This may be carried out by moving the plunger rod in 14 a direction F2 (or proximal direction) extending along the axis X and oriented from the distal end DE toward the proximal end PE. Alternatively, the injection device 10 may be prefilled with the injection fluid and stored while prefilled.

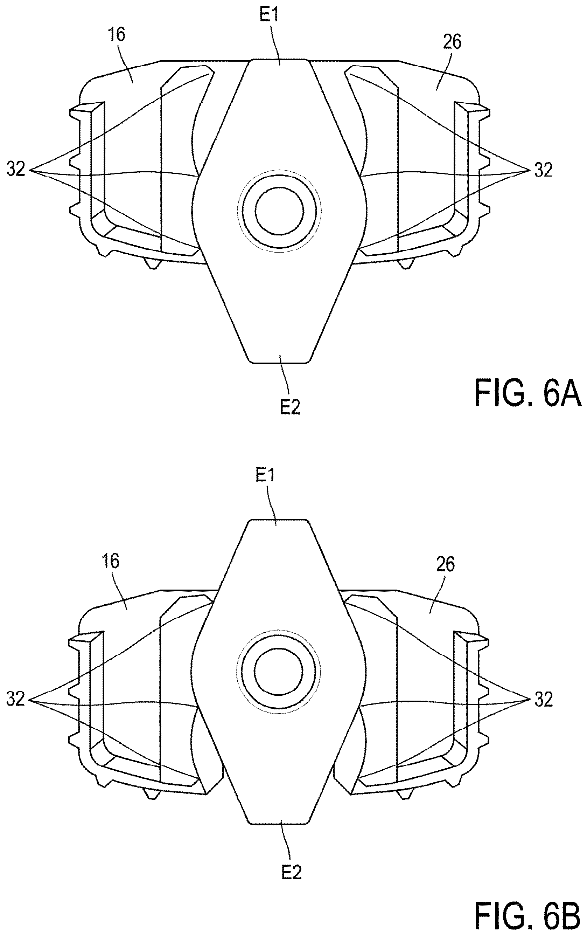

In figure 5B, the dose setting member 16 may be mounted onto the barrel 12 by radially inserting and sliding the flange 24 within the grooves 30 (see arrow I in figures 5B and 6A) up to snap fit the edges E3, E4 with a last pair of reliefs 32, considered in the inserting direction (see fig. 6A). In this position, the 1^{st} position of the dose setting member 16, the 1^{st} portion 16A axially faces the 1^{st} radial projection 14A. A first dose has been then pre-set. As seen in figure 6A, in the 1^{st} position, the tongue E2 extends through the opening 38.

In figure 5C, a first dose, in the present example a priming dose used to expel air bubbles potentially contained into the injection device, is carried out by moving the plunger rod 14 in the first direction F1, as shown by the arrow II, up to abutting the 1^{st} radial projection 14A against the 1^{st} portion 16A.

In Figure 5D, the dose setting member 16 is then radially moved and slid along the second axis X2 (see arrow III), from the 1^{st} position to the 2^{nd} position of the dose setting member 16 in order to pre-set a second dose, corresponding to the first dose to be injected. In this position, the 2^{nd} portion 16B axially faces the 2^{nd} radial projection 14B. As shown in figure 6B, in such a position, the edges E3, E4 are snap fitted with the first pair of reliefs 32, considered in the inserting direction.

In figure 5E, a first injection is carried out by moving the plunger rod 14 in the first direction F1, as shown by the arrow IV, up to abutting the 2^{nd} radial projection 14B against the 2^{nd} portion 16B. It is noted that since the radius of the 1^{st} radial projection 14A is smaller than the minimal radial length RL2 of the 2^{nd} portion 16B, the 1^{st} radial projection does not cooperate with the 2^{nd} portion 16B.

In figure 5F, when the first injection is completed, the dose setting member 16 is radially moved and slid along the second axis X2 (see arrow V), up to be removed from the barrel 12. A 3^{rd} dose is then pre-set, corresponding to the second dose to be injected. In other words, the dose setting member is removed for setting the last dose.

In figure 5G, a second injection is carried out by moving the plunger rod 14 in the first direction F1, as shown by the arrow VI, up to the abutment of a portion of the plunger rod 14 with a portion of the barrel 12.

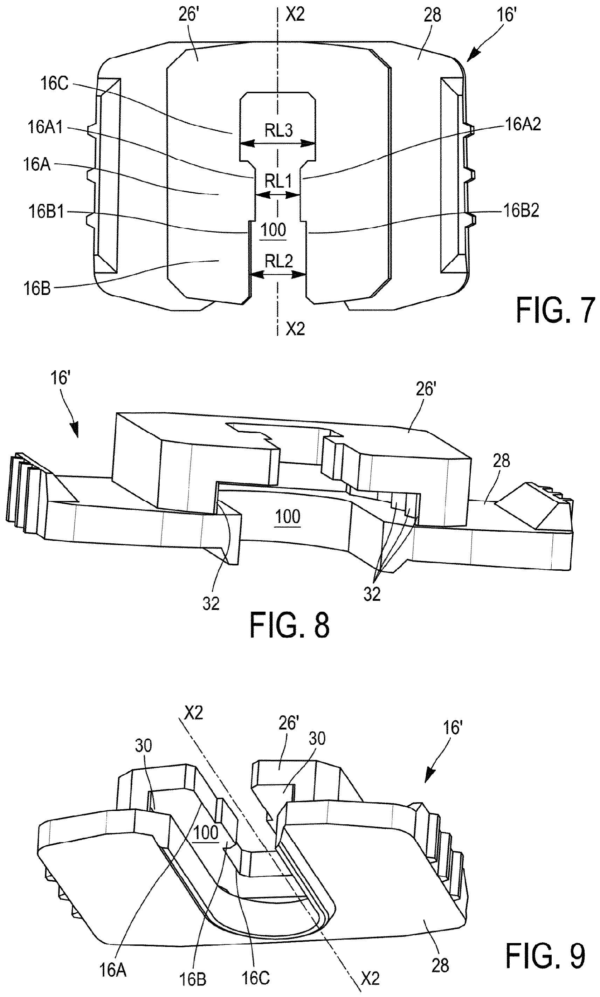

A first variant of the dose setting member 16' is described with reference to figures 7 to 9. The dose setting member 16' is configured to adopt one N+1 position, wherein the plunger rod 14 is axially free relative to the dose setting member 16'.

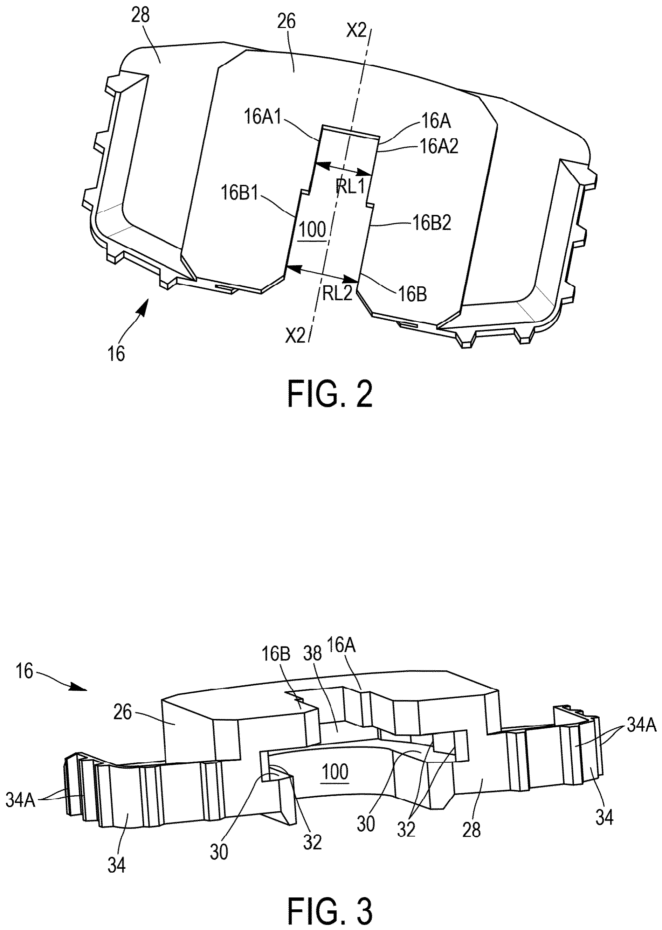

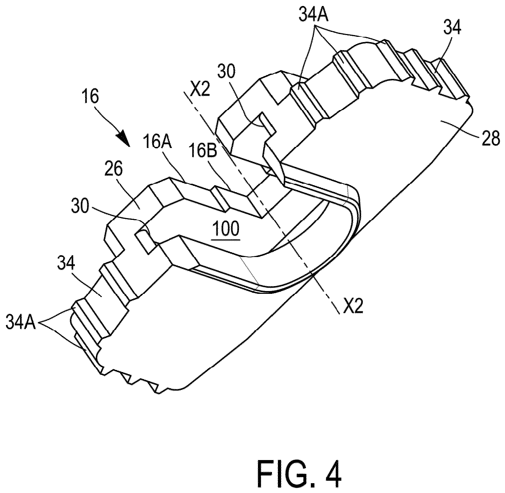

The dose setting member 16' according to the first variant is similar to the dose setting member 16 of figures 2 to 4, except with regard to the grip portion, which is arranged only on the two edges parallel to the second direction X2, and to a wide portion 16C of the notch 100 in the top portion 26', which is not provided on the dose setting member 16 of figures 2 to 4. The other parts are identical or very similar, are not described again and keep the same reference signs. It is note that the wording "wide" is used relative to the other portions, the minimal radial length of the wide portion 16C being greater than the radial length of the other portions 16A, 16C.

In the present example, due to the wide portion 16C the dose setting member 16' defines an axial passage having a minimal radial length RL3 which is greater than the radius of the N^{th} radial projection 14B. The wide portion 16C corresponds to the N+1 position of the dose setting member 16'. In comparison with the dose setting member 16, the first variant 16' is also provided with two additional reliefs 32 (i.e. an additional pair of facing reliefs 32), allowing a snap fitting with the flange 24 in the N+1 position. In the present example, the wide portion 16C is placed before the 1^{st} portion 16A, and corresponds to an initial position or a 0 position. The wide portion 16C is closer to the closed bottom of the "U" than the 1^{st} portion 16A. The 1^{st} portion 16A is axially disposed between the wide portion 16C and the 2^{nd} portion 16B.

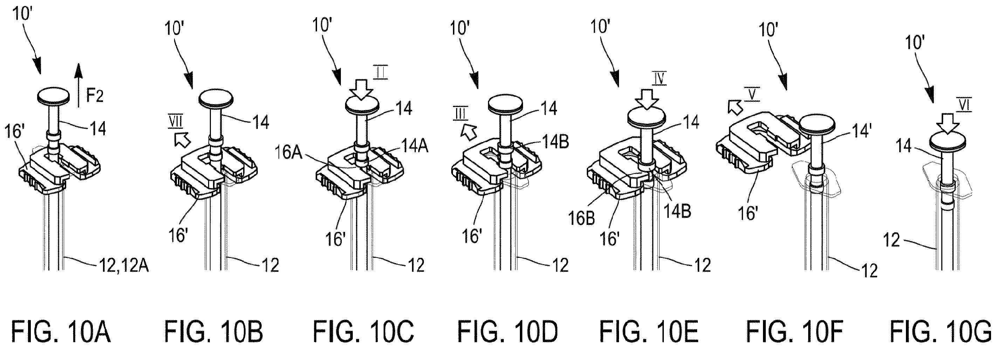

The injection device 10' shown if figures 10A to 10G corresponds to the injection device 10 wherein the dose setting member 16 is replaced by the dose setting member 16' according to the first variant.

A dose setting method for pre-setting a dose of the injection device 10' provided before injection, comprising moving the dose setting member 16' in any one of the N positions so that an n^{th} radial projection axially face an n^{th} portion, is now described with reference to figures 10A to 10G.

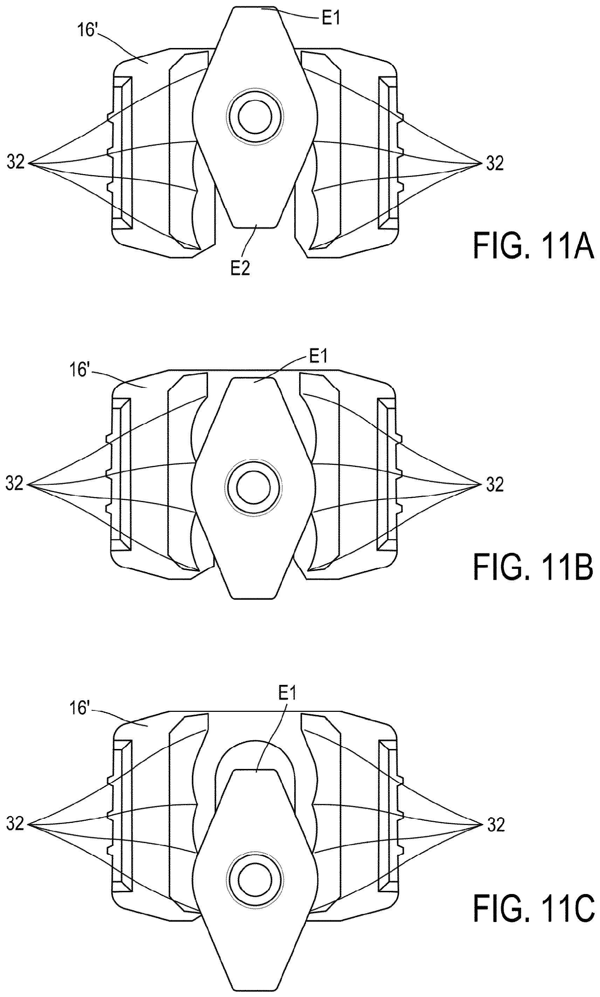

In figure 10A, the dose setting member 16' is mounted (or pre-mounted in comparison with figure 5A) to the barrel 12, and place in the N+1 or initial position (see also the corresponding snap fitting configuration shown in figure 11A). In such a position, no portion of the dose setting member 16' axially faces the radial projections 14A, 14B, and the plunger rod 14 is axially free relative to the dose setting member 16'. A reconstitution of an injection fluid, may be carried out by mixing a prefilled powder and a fluid. As an alternative, an injection fluid may be pumped/sucked from an external vial not shown while the reservoir 12A is initially empty. This may be carried out by moving the plunger rod in 14 a direction F2 extending along the axis X and oriented from the distal end DE toward the proximal end PE. Alternatively, the injection device 10 may be prefilled with the injection fluid and stored while prefilled. In other words, figure 10A correspond to a step of moving the dose setting member 16' in the N+1 position before setting the first dose for filling the injection device 10'.

In figure 10B, the dose setting member 16' may be radially moved and slid along the second axis X2 (see arrow VII) up to snap fit the edges E3, E4 with a second or intermediate pair of reliefs 32 (see fig. 11B). In this position, the 1^{st} position of the dose setting member 16', the 1^{st} portion 16A axially faces the 1^{st} radial projection 14A. A first dose has been then pre-set.

Figures 10C to 10G are strictly similar and strictly correspond to the steps of figures 5C to 5G, respectively, and are not further described. In the same way, figures 11B and 11C respectively corresponds to figures 6A and 6B and are not further described.

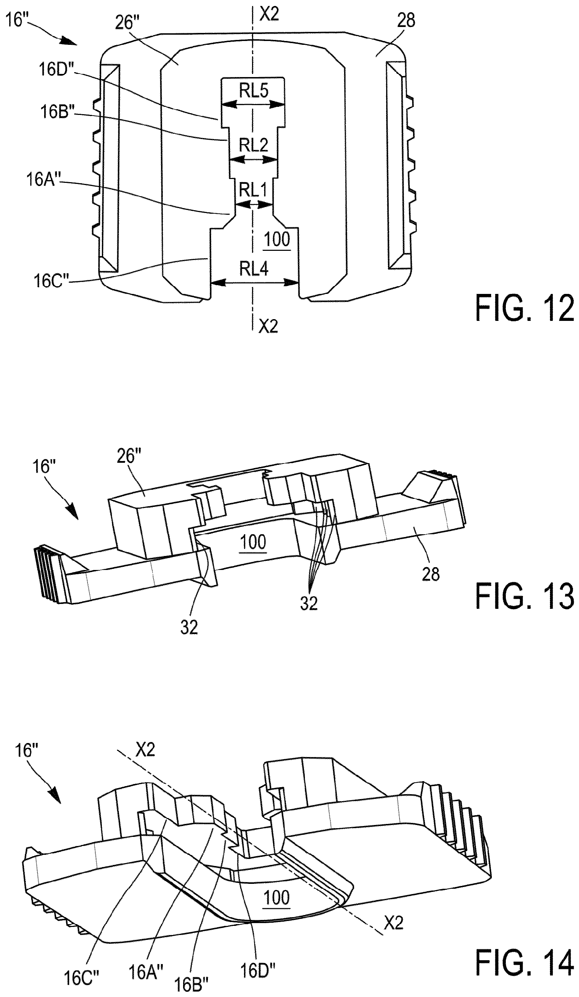

A second variant of the dose setting member 16" is described with reference to figures 12 to 14. The dose setting member 16" is configured to adopt two N+1 positions, wherein the plunger rod 14 is axially free relative to the dose setting member 16". The dose setting member 16" may be un-removable, but not necessarily.

The dose setting member 16" according to the second variant is similar to the dose setting member 16' of the first variant, except with regard to arrangement of the portions on the top portion 26". The other parts are identical or very similar, are not described again and keep the same reference signs.

The top portion 26" of the dose setting member 16" successively comprises, along the second axis X2, a first N+1 portion or wide portion 16C", a 1^{st} portion 16A", a 2^{nd} potion 16B" and a second N+1 portion or wide portion 16D", the first N+1 portion 16C" being closer to the open top of the "U" than the second N+1 portion or wide portion 16D". The second N+1 portion 16D" is closer to the closed bottom of the "U" than the first N+1 portion 16C".

In the present example, due to the first wide portion 16C" the dose setting member 16" defines a first axial passage having a minimal radial length RL4 which is greater than the radius of the N^{th} radial projection 14B. The first wide portion 16C" corresponds to a first N+1 position of the dose setting member 16". Due to the second wide portion 16D", the dose setting member 16" defines a second axial passage having a minimal radial length RL5 which is greater than the radius of the N^{th} radial projection 14B. The second wide portion 16D" corresponds to a second N+1 position of the dose setting member 16". In the present example RL4 is larger than RL5. However, RL4 may be smaller than RL5, or RL4 may be equal to RL5.

In comparison with the dose setting member 16, the second variant 16" is also provided with four additional reliefs 32 (i.e. two additional pairs of facing reliefs 32), allowing a snap fitting with the flange 24 in the first and in the second N+1 positions.

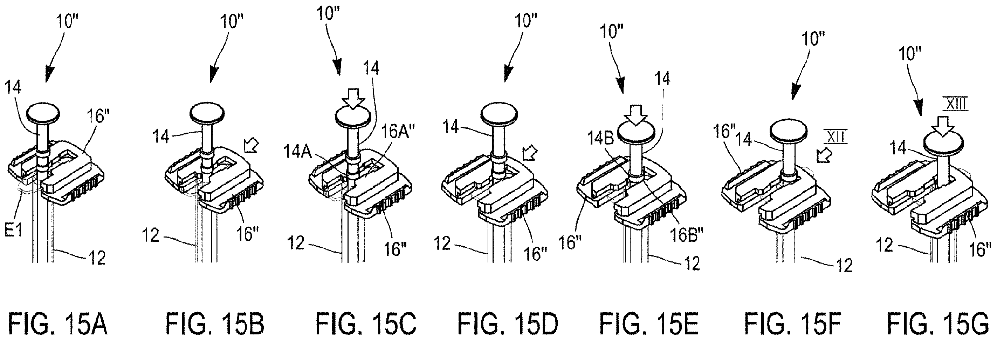

The injection device 10" shown on figures 15A to 15G correspond to the injection device 10 wherein the dose setting member 16 is replaced by the dose setting member 16" according to the second variant.

A dose setting method for pre-setting a dose of the injection device 10" provided before injection, comprising moving the dose setting member 16" in any one of the N positions so that an n^{th} radial projection axially face an n^{th} portion, is now described with reference to figures 15A to 15G.

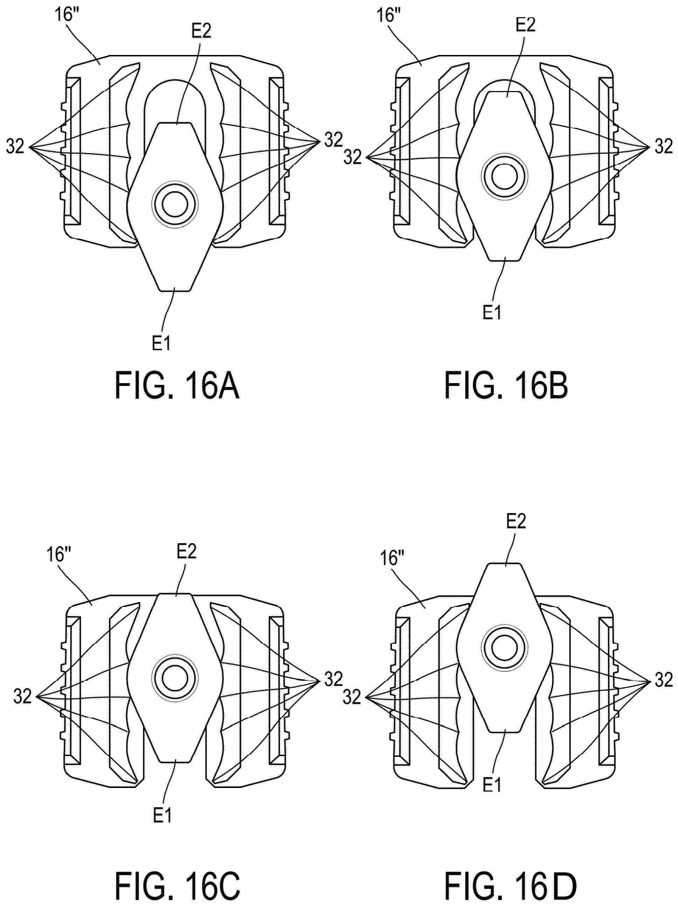

Figure 15A to 15E respectively correspond to figures 10A to 10E, except the radial movement of the dose setting member 16" which is opposed due to the reverse order of the portions along the second axis X2. Therefore, figures 15A to 15E are not further described. In the same way figures 16A to 16C respectively corresponds to figures 11A to 11C and are not further described.

In figure 15F, the dose setting member 16" is moved and slid radially from the 2^{nd} position to the second N+1 position (see arrow XII). The corresponding snap fitting position is shown in figure 16D. This position is the last position of the dose setting member 16", wherein a last dose is pre-set. In other words, figure 15 corresponds to moving the dose setting member 16" in the second N+1 position after completion of the N^{th} dose to allow emptying the injection device 10".

In figure 15G, a last injection is carried out by moving the plunger rod 14 in the first direction F1, as shown by the arrow XIII, up to the abutment of a portion of the plunger rod 14 with a portion of the barrel 12.

Although the present disclosure is described with reference to specific examples, it is to be understood that these examples are merely illustrative of the principles and applications of the present disclosure. In particular, individual characteristics of the various embodiments shown and/or mentioned may be combined in additional embodiments. Consequently, the description and the drawings should be considered in a sense that is illustrative rather than restrictive.

Additionally, all of the disclosed features of an apparatus may be transposed, alone or in combination, to a method and vice versa.

It is intended that the specification and the examples be considered as exemplary only, with a true scope of the invention being indicated by the following claims.

## Claims

1. An injection device (10, 10', 10") including:
- a barrel (12) extending along an axis (X) and defining a reservoir (12A), the barrel (12) comprising a proximal end (PE) and a distal end (DE) having an outlet (22),
- a plunger rod (14) received within the barrel (12) via the proximal end (PE) and configured to move along the axis (X) relative to the barrel (12), the plunger rod (14) comprising N radial projections (14A, 14B) successively disposed at different locations along the axis (X), N being an integer equal or greater than 2,
- a dose setting member (16, 16', 16") configured to be mounted onto the barrel (12), and having N portions (16A, 16B) configured to sequentially abut axially against the N radial projections (14A, 14B), respectively, when moving the plunger rod (14) axially in a first direction (F1) oriented from the proximal end (PE) toward the distal end (DE),
- the dose setting member (16, 16', 16") being configured to be moved relative to the barrel (12) between N positions, wherein the n^{th} radial projection (14A, 14B) axially faces the n^{th} portion (16A, 16B) in the n^{th} position, n being an integer lying between 1 and N,
the injection device (10, 10', 10") being **characterized in that** the dose setting member (16, 16') is removably mounted onto the barrel, the dose setting member (16, 16', 16") comprising a notch (100) extending radially and radially opened, considered when the dose setting member (16, 16', 16") is mounted onto the barrel (12), and configured to receive the barrel (12), and **in that** the barrel (12) comprises a flange (24) extending radially, the dose setting member (16, 16', 16") being configured to be slidably mounted onto the flange (24).

2. The injection device (10, 10', 10") according to claim 1, configured so that the n^{th} portion (16A, 16B) is able to abut only against the n^{th} radial projection (14A, 14B).

3. The injection device (10', 10") according to claim 1 or 2, wherein the dose setting member (16', 16") is configured to adopt at least one N+1 position, wherein the plunger rod (14) is axially free relative to the dose setting member (16', 16").

4. The injection device (10, 10', 10") according to any one of claims 1 to 3, wherein the dose setting member (16, 16', 16") is configured to be radially moved relative to the barrel (12) between N positions.

5. The injection device (10, 10', 10") according to any one of claims 1 to 4, wherein the notch (100) extends along a second axis (X2), the N portions (16A, 16B) being successively disposed at different locations along the second axis (X2).

6. The injection device (10, 10', 10") according to any one of claims 1 to 5, wherein a radial extension of the t^{th} radial projection (14B) is greater than a radial extension of the (t-1)^{th} radial projection (14A) and lower than a radial extension of the (t+1)^{th} radial projection, if any, t being an integer lying between 2 and N.

7. The injection device (10, 10', 10") according to any one of claims 1 to 6, wherein each of the radial projections (14A, 14B) has an annular shape having a radius, the radius of the t^{th} radial projection (14B) being greater than the radius of the (t-1)^{th} radial projection (14A), and lower than the radius of the (t+1)^{th} radial projection, if any, and each of the portions (16A, 16B) defines an axial passage having a minimal radial length, the minimal radial length (RL2) of the t^{th} portion (16B) being greater than minimal radial length (RL1) of the (t-1)^{th} portion (16A) and lower than minimal radial length of the (t+1)^{th} portion, if any, t being an integer lying between 2 and N.

8. The injection device (10', 10") according to claims 3 and 7, wherein in the at least one N+1 position, the dose setting member (16', 16") defines an axial passage having a minimal radial length (RL3, RL4, RL5) which is greater than the radius of the N^{th} radial projection (14B).

9. The injection device (10, 10', 10") according to any one of claims 6 to 8, wherein the (t-1)^{th} radial projection (14A) is axially closer to the distal end (DE) than the t^{th} radial projection (14B).

10. The injection device (10, 10', 10") according to any one of claims 1 to 9, wherein the flange (24) comprises a guiding portion (GP) configured to slidably guide the dose setting member (16, 16', 16"), and a retaining portion (RP) configured to maintain the dose setting member (16, 16', 16") in any of the N positions.

11. A dose setting method for pre-setting a dose of an injection device before injection, the dose setting method comprising providing an injection device (10, 10', 10") according to any one of claims 1 to 10, and moving the dose setting member in any one of the N positions so that a n^{th} radial projection axially face a n^{th} portion.

12. The dose setting method according to claim 11, wherein the dose setting member (16, 16') is removably mounted onto the barrel, the dose setting method comprising removing the dose setting member (16, 16') for setting the last dose.

## Patentansprüche

1. Einspritzvorrichtung (10, 10', 10"), die Folgendes enthält:
- einen Zylinder (12), der sich entlang einer Achse (X) erstreckt und ein Reservoir (12A) definiert, wobei der Zylinder (12) ein proximales Ende (PE) und ein distales Ende (DE) umfasst, das einen Auslass (22) aufweist,
- eine Kolbenstange (14), die über das proximale Ende (PE) innerhalb des Zylinders (12) aufgenommen wird und so eingerichtet ist, dass sie sich entlang der Achse (X) relativ zum Zylinder (12) bewegt, wobei die Kolbenstange (14) N radiale Vorsprünge (14A, 14B) umfasst, die nacheinander an verschiedenen Stellen entlang der Achse (X) angeordnet sind, wobei N eine Ganzzahl ist, die gleich oder größer ist als 2,
- ein Dosiseinstellungselement (16, 16', 16"), das so eingerichtet ist, dass es auf dem Zylinder (12) montiert ist, und N Abschnitte (16A, 16B) aufweist, die so eingerichtet sind, dass sie sequenziell axial gegen die N radialen Vorsprünge (14A, 14B) anliegen, wenn die Kolbenstange (14) axial in eine erste Richtung (F1) bewegt wird, die vom proximalen Ende (PE) in Richtung des distalen Endes (DE) ausgerichtet ist,
- wobei das Dosiseinstellungselement (16, 16', 16") so eingerichtet ist, dass es relativ zum Zylinder (12) zwischen N Positionen bewegt werden kann, wobei der n-te radiale Vorsprung (14A, 14B) axial dem n-ten Abschnitt (16A, 16B) in der n-ten Position zugewandt ist, wobei n eine Ganzzahl ist, die zwischen 1 und N liegt,
wobei die Einspritzvorrichtung (10, 10', 10'') **dadurch gekennzeichnet ist, dass** das Dosiseinstellungselement (16, 16') abnehmbar auf dem Zylinder montiert ist, wobei das Dosiseinstellungselement (16, 16', 16") eine radial erstreckende und radial geöffnete Kerbe (100) umfasst, unter Berücksichtigung, dass das Dosiseinstellungselement (16, 16', 16") auf dem Zylinder (12) montiert ist und so eingerichtet ist, dass es den Zylinder (12) aufnimmt, und dass der Zylinder (12) einen radial erstreckenden Flansch (24) umfasst, wobei das Dosiseinstellungselement (16, 16', 16") eingerichtet ist, um verschiebbar auf dem Flansch (24) montiert zu sein.

2. Einspritzvorrichtung (10, 10', 10") nach Anspruch 1, die so eingerichtet ist, dass der n-te Abschnitt (16A, 16B) nur gegen den n-ten radialen Vorsprung (14A, 14B) anliegen kann.

3. Einspritzvorrichtung (10', 10'') nach Anspruch 1 oder 2, wobei das Dosiseinstellungselement (16', 16") so eingerichtet ist, dass es mindestens eine Position N+1 einnimmt, wobei die Kolbenstange (14) relativ zum Dosiseinstellungselement (16', 16") axial frei ist.

4. Einspritzvorrichtung (10, 10', 10'') nach einem der Ansprüche 1 bis 3, wobei das Dosiseinstellungselement (16, 16', 16") so eingerichtet ist, dass es relativ zum Zylinder (12) zwischen N Positionen radial verschoben wird.

5. Einspritzvorrichtung (10, 10', 10'') nach einem der Ansprüche 1 bis 4, wobei sich die Kerbe (100) entlang einer zweiten Achse (X2) erstreckt, wobei die N Abschnitte (16A, 16B) nacheinander an verschiedenen Stellen entlang der zweiten Achse (X2) angeordnet sind.

6. Einspritzvorrichtung (10, 10', 10'') nach einem der Ansprüche 1 bis 5, wobei eine radiale Verlängerung des t-ten radialen Vorsprungs (14B) größer als eine radiale Verlängerung des (t-1)-ten radialen Vorsprungs (14A) und kleiner ist als eine radiale Verlängerung des (t+1)-ten radialen Vorsprungs, falls vorhanden, wobei n eine Ganzzahl ist, die zwischen 2 und N liegt.

7. Einspritzvorrichtung (10, 10', 10'') nach einem der Ansprüche 1 bis 6, wobei jeder der radialen Vorsprünge (14A, 14B) eine ringförmige Form aufweist, die einen Radius aufweist, wobei der Radius des t-ten radialen Vorsprungs (14B) größer als der Radius des (t-1)-ten radialen Vorsprungs (14A) und kleiner ist als der Radius des (t+1)-ten radialen Vorsprungs, falls vorhanden, und jeder der Abschnitte (16A, 16B) einen axialen Durchgang mit einer minimalen radialen Länge definiert, wobei die minimale radiale Länge (RL2) des t-ten Abschnitts (16B) größer als die minimale radiale Länge (RL1) des (t-1)-ten Abschnitts (16A) und kleiner ist als die minimale radiale Länge des (t+1)-ten Abschnitts, falls vorhanden, wobei n eine Ganzzahl ist, die zwischen 2 und N liegt.

8. Einspritzvorrichtung (10', 10'') nach den Ansprüchen 3 und 7, wobei in der mindestens einen Position N+1 das Dosiseinstellungselement (16', 16") einen axialen Durchgang definiert, der eine minimale radiale Länge (RL3, RL4, RL5) aufweist, die größer ist als der Radius des N-ten radialen Vorsprungs (14B).

9. Einspritzvorrichtung (10, 10', 10'') nach einem der Ansprüche 6 bis 8, wobei der (t-1)-te radiale Vorsprung (14A) axial näher am distalen Ende (DE) liegt als der t-te radiale Vorsprung (14B).

10. Einspritzvorrichtung (10, 10', 10'') nach einem der Ansprüche 1 bis 9, wobei der Flansch (24) einen Führungsabschnitt (GP) umfasst, der so eingerichtet ist, dass er das Dosiseinstellungselement (16, 16', 16") gleitend führt, sowie einem Halteabschnitt (RP), der so eingerichtet ist, dass er das Dosiseinstellungselement (16, 16', 16") in einer der N Positionen hält.

11. Dosiseinstellungsverfahren zum Voreinstellen einer Dosis einer Einspritzvorrichtung vor dem Einspritzen, wobei das Dosiseinstellungsverfahren das Bereitstellen einer Einspritzvorrichtung (10, 10', 10'') nach einem der Ansprüche 1 bis 10 umfasst, und das Dosiseinstellungselement in eine der N Positionen so verschiebt, dass eine n-te radiale Projektion axial einem n-ten Abschnitt zugewandt ist.

12. Dosiseinstellungsverfahren nach Anspruch 11, wobei das Dosiseinstellungselement (16, 16') abnehmbar auf dem Zylinder montiert ist, wobei das Dosiseinstellungsverfahren das Entfernen des Dosiseinstellungselements (16, 16') zum Einstellen der letzten Dosis umfasst.

## Revendications

1. Dispositif d'injection (10, 10', 10") comprenant :
- un cylindre (12) s'étendant le long d'un axe (X) et définissant un réservoir (12A), le cylindre (12) comprenant une extrémité proximale (PE) et une extrémité distale (DE) ayant une sortie (22),
- une tige de piston (14) reçue à l'intérieur du cylindre (12) par l'extrémité proximale (PE) et configurée pour se déplacer le long de l'axe (X) par rapport au cylindre (12), la tige de piston (14) comprenant N saillies radiales (14A, 14B) disposées successivement à différents emplacements le long de l'axe (X), N étant un nombre entier supérieur ou égal à 2,
- un élément de réglage de dose (16, 16', 16") configuré pour être monté sur le cylindre (12), et ayant N parties (16A, 16B) configurées pour venir en butée axialement, de manière séquentielle, contre les N saillies radiales (14A, 14B), respectivement, lors du déplacement axial de la tige de piston (14) dans une première direction (F1) orientée de l'extrémité proximale (PE) vers l'extrémité distale (DE),
- l'élément de réglage de dose (16, 16', 16") étant configuré pour être déplacé par rapport au cylindre (12) entre N positions, où la n-ième saillie radiale (14A, 14B) est tournée axialement vers la n-ième partie (16A, 16B) dans la n-ième position, n étant un nombre entier compris entre 1 et N,
le dispositif d'injection (10, 10', 10") étant **caractérisé en ce que** l'élément de réglage de dose (16, 16') est monté de manière amovible sur le cylindre, l'élément de réglage de dose (16, 16', 16") comprenant une encoche (100) s'étendant radialement et ouverte radialement, lorsque l'élément de réglage de dose (16, 16', 16") est monté sur le cylindre (12), et configurée pour recevoir le cylindre (12), et **en ce que** le cylindre (12) comprend une bride (24) s'étendant radialement, l'élément de réglage de dose (16, 16', 16") étant configuré pour être monté en coulissement sur la bride (24).

2. Dispositif d'injection (10, 10', 10") selon la revendication 1, configuré de sorte que la n-ième partie (16A, 16B) ne puisse venir en butée que contre la n-ième saillie radiale (14A, 14B).

3. Dispositif d'injection (10', 10") selon la revendication 1 ou 2, dans lequel l'élément de réglage de dose (16', 16") est configuré pour adopter au moins une position N+1, dans lequel la tige de piston (14) est libre axialement par rapport à l'élément de réglage de dose (16', 16'').

4. Dispositif d'injection (10, 10', 10") selon l'une quelconque des revendications 1 à 3, dans lequel l'élément de réglage de dose (16, 16', 16") est configuré pour être déplacé radialement par rapport au cylindre (12) entre N positions.

5. Dispositif d'injection (10, 10', 10") selon l'une quelconque des revendications 1 à 4, dans lequel l'encoche (100) s'étend le long d'un second axe (X2), les N parties (16A, 16B) étant disposées successivement à différents emplacements le long du second axe (X2).

6. Dispositif d'injection (10, 10', 10") selon l'une quelconque des revendications 1 à 5, dans lequel une extension radiale de la t-ième saillie radiale (14B) est supérieure à une extension radiale de la (t-1)-ième saillie radiale (14A) et inférieure à une extension radiale de la (t+1)-ième saillie radiale, le cas échéant, t étant un nombre entier compris entre 2 et N.

7. Dispositif d'injection (10, 10', 10") selon l'une quelconque des revendications 1 à 6, dans lequel chacune des saillies radiales (14A, 14B) a une forme annulaire ayant un rayon, le rayon de la t-ième saillie radiale (14B) étant supérieur au rayon de la (t-1)-ième saillie radiale (14A) et inférieur au rayon de la (t+1)-ième saillie radiale, le cas échéant, et chacune des parties (16A, 16B) définit un passage axial ayant une longueur radiale minimale, la longueur radiale minimale (RL2) de la t-ième partie (16B) étant supérieure à la longueur radiale minimale (RL1) de la (t-1)-ième partie (16A) et inférieure à la longueur radiale minimale de la (t+1)-ième partie, le cas échéant, t étant un nombre entier compris entre 2 et N.

8. Dispositif d'injection (10', 10") selon les revendications 3 et 7, dans lequel, dans l'au moins une position N+1, l'élément de réglage de dose (16', 16") définit un passage axial ayant une longueur radiale minimale (RL3, RL4, RL5) qui est supérieure au rayon de la N-ième saillie radiale (14B).

9. Dispositif d'injection (10, 10', 10") selon l'une quelconque des revendications 6 à 8, dans lequel la (t-1)-ième saillie radiale (14A) est axialement plus proche de l'extrémité distale (DE) que la t-ième saillie radiale (14B).

10. Dispositif d'injection (10, 10', 10") selon l'une quelconque des revendications 1 à 9, dans lequel la bride (24) comprend une partie de guidage (GP) configurée pour guider en coulissement l'élément de réglage de dose (16, 16', 16''), et une partie de retenue (RP) configurée pour maintenir l'élément de réglage de dose (16, 16', 16") dans l'une des N positions.

11. Procédé de réglage de dose pour prérégler une dose d'un dispositif d'injection avant l'injection, le procédé de réglage de dose comprenant la fourniture d'un dispositif d'injection (10, 10', 10") selon l'une quelconque des revendications 1 à 10, et le déplacement de l'élément de réglage de dose dans l'une quelconque des N positions de sorte qu'une n-ième saillie radiale soit tournée axialement vers une n-ième partie.

12. Procédé de réglage de dose selon la revendication 11, dans lequel l'élément de réglage de dose (16, 16') est monté de manière amovible sur le cylindre, le procédé de réglage de dose comprenant le retrait de l'élément de réglage de dose (16, 16') pour régler la dernière dose.
